# EUROPEAN PATENT APPLICATION

(11) **EP 0 729 752 A2**
(43) Date of publication of application: **04.09.1996**
(21) Application number: 96200242.4
(22) Date of filing: 20.12.1991
(51) Int. Cl.: A61K 31/195, A61K 31/40, A61K 31/415, A61K 31/445, A61K 31/505, A61K 31/535, A61K 31/55

(54) **Central cholecystokinin antagonists having pharmaceutical activity**

(62) Divisional of application: 92903432.0
(71) Applicant: MERCK SHARP & DOHME LTD., Hoddesdon Hertfordshire EN11 9BU (GB)
(72) Inventor: Horwell, Davis Christopher, Foxton, Cambridge (GB); Hughes, John, Swaffman Prior, Cambridge CB5 0HT (GB); Woodruff, Geoffrey Neil, Braughing, Nr. Ware, Hertfordshire (GB)
(74) Representative: Thompson, John Dr.

(57) **Abstract**

Pharmaceutical compositions and methods of using CCK-ligands as antipsychotic, antianxiety, and agents useful in treatment or prevention of withdrawal symptoms caused by withdrawal of chronic or long-term use of diazepam, alcohol, cocaine, or nicotine, and antianxiety agents are described.

## Description

### BACKGROUND OF THE INVENTION

Agents acting at central cholecystokinin (CCK) receptors induce satiety (Schick, Yaksh, and Go, Regulatory Peptides 14:277-291, 1986. They are also expected to act as analgesics (Hill, Hughes, and Pittaway, Neuropharmacology 26:289-300, 1987, and as anticonvulsants (MacVicar, Kerrin, and Davison, Brain Research 406:130-135, 1987.

Reduced levels of CCK-peptides have been found in the brains of schizophrenic patients compared with controls (Roberts, Ferrier, Lee, Crow, Johnstone, Owens, Bacarese-Hamilton, McGregor, O'Shaughnessey, Polak, and Bloom, Brain Research 288:199-211, 1983). It has been proposed that changes in the activity of CCK neurones projecting to the nucleus accumbens may play a role in schizophrenic processes by influencing dopaminergic function (Totterdell and Smith, Neuroscience 19:181-192, 1986). This is consistent with numerous reports that CCK peptides modulate dopaminergic function in the basal ganglia and particularly the nucleus accumbens (Weiss, Tanzer, and Ettenberg, Pharmacology, Biochemistry and Behaviour 30:309-317, 1988; Schneider, Allpert, and Iversen, Peptides 4:749-753, 1983). It may therefore be expected that agents modifying CCK receptor activity may have therapeutic value in conditions associated with disturbed function of central dopaminergic function such as schizophrenia and Parkinson's disease.

CCK and gastrin peptides share a common carboxy terminal pentapeptide sequence and CCK peptides can bind to the gastrin receptor of the stomach mucosa and elicit acid secretion in many species including human (Konturek, Gastrointestinal Hormones, Ch. 23, pp. 529-564, 1980, G. G. J. Glass, ed., Raven Press, NY). Antagonists of the CCK-8 receptor would also be expected to be antagonists at the stomach gastrin receptor and thus be of value for conditions involving excessive acid secretion.

CCK and gastrin peptides have trophic effects on the pancreas and various tissues of the gastrointestinal tract (Johnson, ibid., pp. 507-527), actions which are associated with increased DNA and RNA synthesis. Moreover, gastrin secreting cells are associated with certain gastrointestinal tumors as in the Zollinger-Ellison syndrome (Stadil, ibid., pp. 279-739), and some colorectal tumors may also be gastrin-/CCK-dependent (Singh, Walker, Townsend, and Thompson, Cancer Research 46:1612, 1986, and Smith, J. P., Gastroenterology 95:1541, 1988). Antagonists of CCK/gastrin receptors could, therefore, be of therapeutic value as antitumor agents.

The cholecystokinin peptides are widely distributed in various organs of the body including the gastrointestinal tract, endocrine glands, and the nerves of the peripheral and central nervous systems. Various biologically active forms have been identified including a 33-amino acid hormone and various carboxyl-terminus fragments of this peptide (e.g., the octapeptide CCK26-33 and the tetrapeptide CCK30-33) (G. J. Dockray, Br. Med. Bull. 38(3):253-258, 1982).

The various CCK peptides are thought to be involved in the control of smooth muscle contractility, exocrine and endocrine gland secretion, sensory nerve transmission, and numerous brain functions. Administration of the native peptides cause gall bladder contraction, amylase secretion, excitation of central neurons, inhibition of feeding, anticonvulsive actions, and other behavioral effects (Cholecystokinin: Isolation, Structure and Functions, G. B. J. Glass, ed., Raven Press, New York, 1980, pp. 169-221; J. E. Morley, Life Sciences 27:355-368, 1980; Cholecystokinin in the Nervous System, J. de Belleroche and G. J. Dockray, eds., Ellis Horwood: Chichester, England, 1980, pp. 110-127).

The high concentrations of CCK peptides in many brain areas also indicate major brain functions for these peptides (G. J. Dockray, Br. Med. Bull. 38(3):253-258, 1982). The most abundant form of brain CCK found is CCK26-33, although small quantities of CCK30-33 exist (Rehfeld and Gotterman, J. Neurochem. 32:1339-1341, 1979). The role of central nervous system CCK is not known with certainty, but it has been implicated in the control of feeding (Della-Fera and Baile, Science 206:471-473, 1979).

CCK is known to be present in some cortical interneurones which also contain gamma-aminobutyric acid (GABA) (H. Demeulemeester, et al, J. Neuroscience 8:988-1000, 1988). Agents that modify GABA action may have utility as anxiolytic or hypnotic agents (S. C. Harvey, The Pharmacological Basis of Therapeutics (7th ed.), MacMillan, 1985, pp. 339-371. Thus, agents which modify CCK action may have parallel anxiolytic or hypnotic activities.

It has now been found that CCK-B ligands are useful as antipsychotic and as antianxiety agents. This includes all CCK-B ligands. CCK-B antagonists have now been shown to be agents useful in the treatment of withdrawal from drugs and alcohol. Although some ligands are CCK-A selective they do have some CCK-B activity as well and therefore are included also. Mixed CCK-A/-B ligands are also included.

The present invention is related to pharmaceutical compositions and methods of treating psychoses and/or anxiety and the withdrawal response caused by chronic treatment or abuse followed by withdrawal of drugs or alcohol using CCK-ligands and pharmaceutically acceptable salts thereof. The compounds and methods of preparing them are found in United States Patents 4,791,215 and 4,820,834 and European Application 167,919. These documents are incorporated herein by reference.

Other compounds and methods of preparing the are disclosed in J. Med. Chem. 34(4):1505-8, 1991 and in Drugs of the Future 16(7):631-640, 1991.

The uses disclosed are gastric acid secretion disorders, gastrointestinal motility, pancreatic secretions, dopaminergic functions, analgesics, psychic disturbances, anorexia, weight increases in farm animals, and pathological cellular growth such as tumors.

### SUMMARY AND DETAILED DESCRIPTION OF THE INVENTION

The present invention relates to a pharmaceutical composition useful in the treatment of psychoses and/or anxiety. The present invention further relates to a pharmaceutical composition useful for the treatment of withdrawal symptoms, withdrawal from drugs or alcohol. The composition comprises a therapeutically effective amount of at least one of the following CCK-antagonists or a pharmaceutically acceptable salt thereof in combination with a pharmaceutically acceptable carrier.

They are a pharmaceutically active derivative of D,L-glutamic acid and D,L-aspartic acid of formulae: 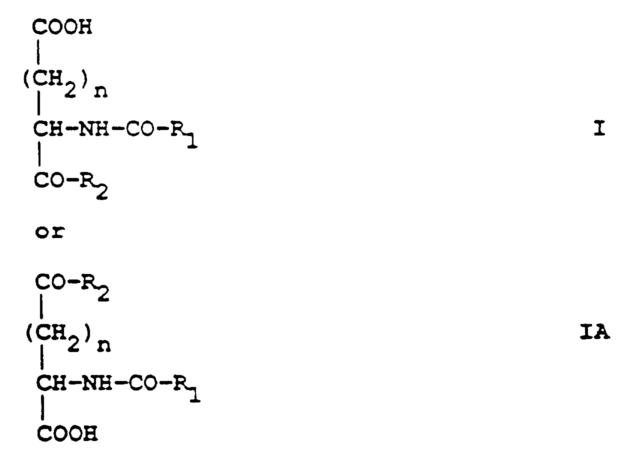
wherein n is 1 or 2;
- R₁ is: a phenyl group mono-, di-, or tri-substituted with linear or branched C₁-C₄ alkyl groups, which may be the same or different, or with halogens, with a cyano group or with a trifluoromethyl group;
- R₂ is: selected from the group consisting of morpholino, piperidino, and amino with one or two linear, branched, or cyclic alkyl group substituents containing from 1 to 8 carbon atoms which may be the same or different; or 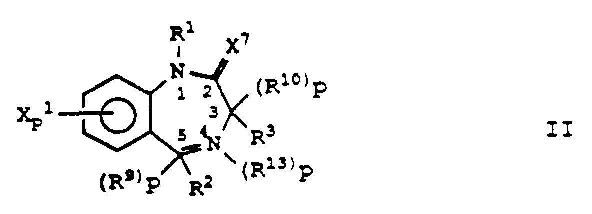
wherein
- R¹ is: H, C₁-C₆ linear or branched alkyl, loweralkenyl, lower alkynyl, -X¹²COOR⁶, -X¹¹cycloloweralkyl, -X¹²NR⁴R⁵, -X¹²CONR⁴R⁵, -X¹²CN, or -X¹¹CX₃¹⁰;
- R² is: H, loweralkyl, substituted or unsubstituted phenyl (wherein the substituents may be 1 or 2 of halo, loweralkyl, loweralkoxy, lowralkylthio, carboxyl, carboxyloweralkyl, nitro, -CF₃, or hydroxy), 2-, 3-, 4-pyridyl, 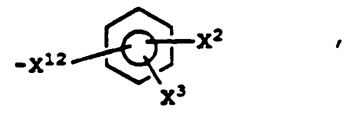 -X¹²SCH₃, -X¹²SOCH₃, -X¹²SO₂CH₃, or -X¹²COOR⁶;
- R³ is: -X¹¹R⁷, 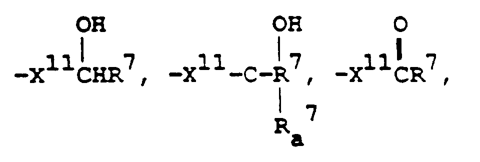 -X¹¹NR¹⁸(CH₂)_{q}R⁷, 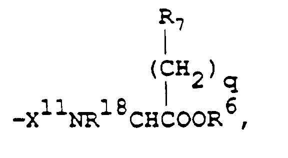 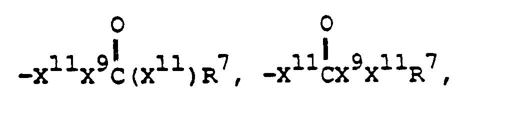 -NH(CH₂)₂₋₃NHR⁷, -NH(CH₂)₂₋₃NHCOR⁷, 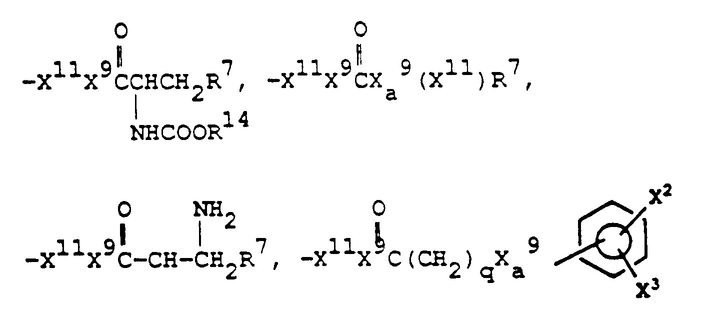 -X¹¹NR¹⁸SO₂(CH₂)_{q}R⁷ or 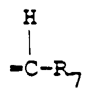
wherein
- R₄ and R₅: are independently R⁶ or in combination with the N of the NR⁴R⁵ group form an unsubstituted or mono or disubstituted, saturated or unsaturated, 4- to 7-membered heterocyclic ring or benzofused 4- to 7-membered heterocyclic ring, or said heterocyclic ring or said benzofused heterocyclic ring which further comprises a second heteroatom selected from O and NCH₃ and the substituent(s) is/are independently selected from C₁₋₄alkyl;
- R⁶ is: H, loweralkyl, cycloloweralkyl, substituted or unsubstituted phenyl, or substituted or unsubstituted phenylloweralkyl wherein the phenyl or phenylloweralkyl substituents may be 1 or 2 of halo, loweralkyl, loweralkoxy, nitro, or CF₃;
- R₇ and Rₐ⁷: are independently α- or β-naphthyl, substituted or unsubstituted phenyl (wherein the substituents may be 1 or 2 of halo, -NO₂, -OH, -X¹¹NR⁴R⁵, loweralkyl, CF₃, CN, SCF₃, C=CH, CH₂SCF₃, 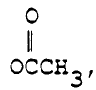 OCHF₂, SH, SPh, PO₃H-loweralkoxy, or loweralkylthio, COOH), 2-, 3-, 4-pyridyl, 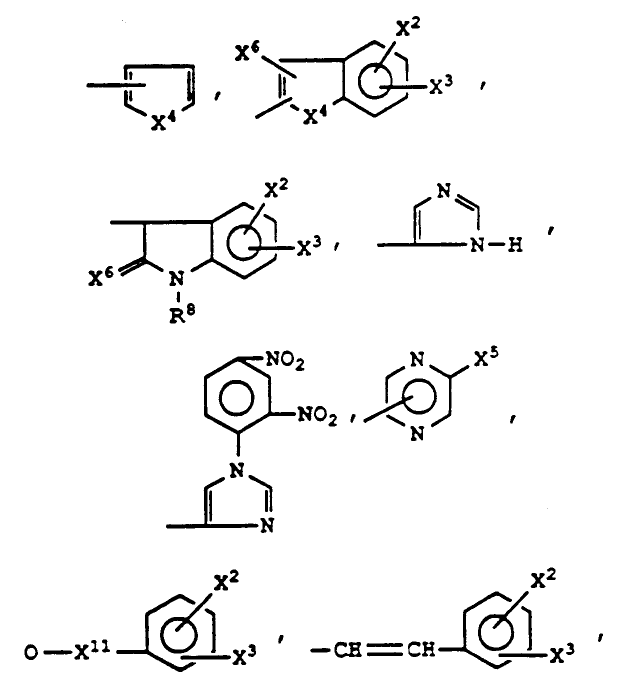 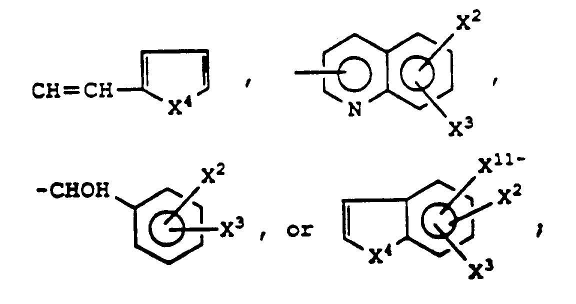
- R⁸ is: H, loweralkyl, cycloloweralkyl, -X¹²CONH₂, -X¹²COOR⁶, -X¹²-cycloloweralkyl, -X¹²NR⁴R⁵, 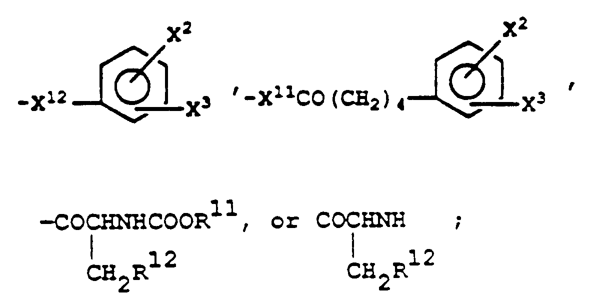
- R⁹ and R¹⁰: are independently H, -OH, or -CH₃,
- R¹¹ and R¹²: are independently loweralkyl or cycloloweralkyl;
- R¹³ is: H, loweralkyl, acyl, O, or cycloloweralkyl;
- R¹⁴ is: loweralkyl or phenylloweralkyl;
- R¹⁵ is: H, loweralkyl, 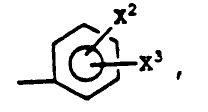 or -NH₂;
- R¹⁸ is: H, loweralkyl, or acyl;
- p is: 0 when its adjacent == is unsaturated and 1 when its adjacent == is saturated except that when R¹³ is O, p is 1 and == is unsaturated;
- q is: 0 to 4;
- r is: 1 or 2;
- X¹ is: H, -NO₂, CF₃, CN, OH, loweralkyl, halo, loweralkylthio, loweralkoxy, -X¹¹COOR⁶, or -X¹¹NR⁴R⁵-;
- X² and X³: are independently H, -OH, -NO², halo, loweralkylthio, loweralkyl, or loweralkoxy;
- X⁴ is: S, O, CH₂, or NR¹⁸ or NR⁸;
- X⁵ is: H, CF₃, CN, -COOR⁶, NO₂, or halo;
- X⁶ is: O or HH;
- X⁷ is: O, S, HH, or NR¹⁵;
- X⁸ is: H or loweralkyl;
- X⁹ and Xₐ⁹: are independently NR¹⁸ or O;
- X¹⁰ is: F, Cl, or Br;
- X¹¹ is: absent or C₁₋₄ linear or branched alkylidene;
- X¹² is: C₁₋₄ linear or branched alkylidene;
- -- is: a saturated or unsaturated bond; or 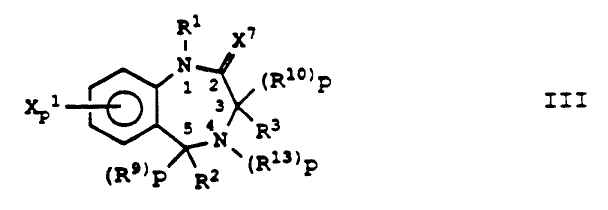
wherein R¹, R², R⁴, R⁵, R⁶, R⁸, R⁹, R¹⁰, R¹¹, R¹², R¹³, R¹⁴, R¹⁵, p, q, r, X¹, X², X³, X⁵, X⁶, X⁷, X⁸, X⁹, X¹⁰, X¹¹, and X¹² are as defined above,
- R³ is: -X¹¹NR¹⁸(CH₂)_{q}R¹⁶, 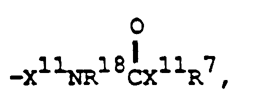 -NH(CH₂)₂₋₃NHR⁷, -NR(CH₂)₂₋₃NHCOR⁷, 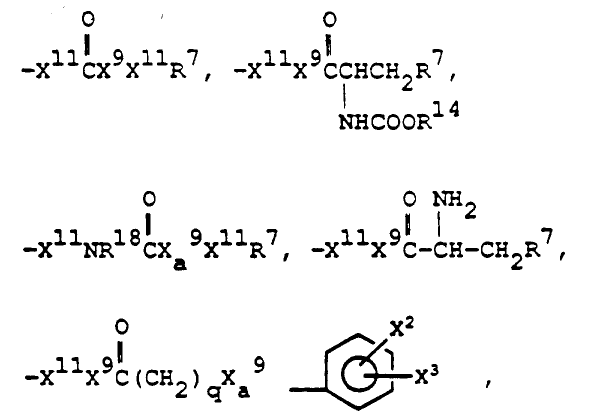 -X¹¹NR¹⁸SO₂(CH₂)_{q}R⁷ or 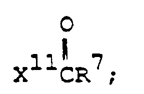
- R⁷ is: α- or β-naphthyl, substituted or unsubstituted phenyl (wherein the substituents may be 1 to 2 of halo, -NO², -OH, -X¹¹NR⁴R⁵, loweralkyl, CF₃, CN, SCF₃, C=CH, CH₂SCF₃, 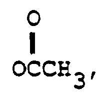 OCHF₂, SH, SPh, PO₃H, loweralkoxy, loweralkylthio, or COOH), 2-, 3-, 4-pyridyl, 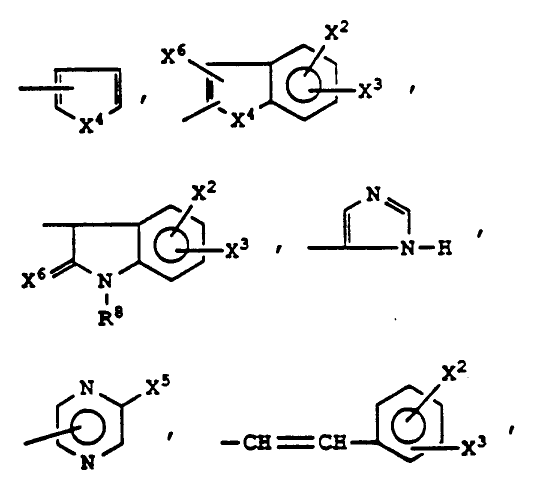 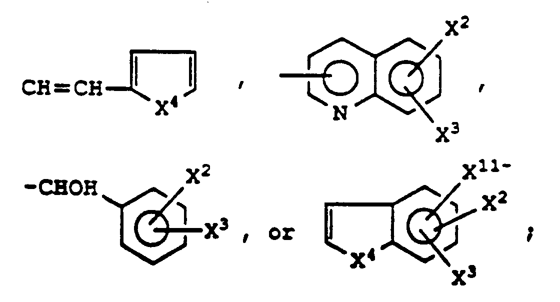 R¹⁶ is α- or β-naphthyl or 2-indolyl;
R¹⁸ is H or loweralkyl; and
= is a saturated or unsaturated bond.

Preferred compounds for use in the pharmaceutical composition of the instant invention are: lorglumide which is DL-4-(3,4-dichlorobenzoylamino)-5-(dipentylamino)-5-oxopentanoic acid, loxiglumide which is (±)-4-[(3,4-dichlorobenzoyl)-amino]-5-[(3-methoxyproxyl)pentylamino]-5-oxo-pentanoic acid, L-364718 which is 3(S)-(-)-N-(2,3-dihydro-1-methyl-2-oxo-5-phenyl-1H-1,4-benzodiazepin-3-yl)-1H-indole-2-carboxamide, and L-365,260 which is (R)-N-(2,3-dihydro-1-methyl-2-oxo-5-phenyl-1H-1,4-benzodiazepine-3-yl)-N'-(3-methylphenyl)urea.

Other preferred compounds for use in the pharmaceutical compositions and methods of treatment of the instant invention are:
Virginiamycins, anthramycin, LY-262,690 which is trans-5-(2-chlorophenyl)-3-oxo-4-phenyl-N-[4-(trifluoromethyl)phenyl]-1-pyrazolidinecarboxamide,
LY-262,691 which is trans-5-(2-chlorophenyl)-3 oxo-4-phenyl-N-[4-(bromo)phenyl]-1-pyrazolidinecarboxamide,
L-365091 which is 1-((3-((((4-chlorophenyl)-amino)carbonyl)amino)-2,3-dihydro-2-oxo-5-phenyl-1H-1,4-benzodiazepin-1-yl)acetyl)pyrrolidine,
(S)-5-[(10,11-dihydrodibenzo[a,d]cyclohepten-5-yl)amino)-4-[(1H-indol-2-yl)carbonyl]amino)-5-oxo-pentanoic acid, and
5,6-dihydro-4-oxo-4H-pyrrolo[1,2-a)thieno[3,2-f][1,4]-diazepin-6-yl)cyanoacetate.

Virginiamycin, and analogs of are disclosed in United States Patents 4,762,923, 4,894,370, 4,772,595, 4,859,690, 4,894,370, 4,942,230, and 5,006,466, all of which are incorporated herein by reference. These list utilities: antimicrobial, analgesic, agents for gastrointestinal uses, central nervous, and regulatory systems.

Still other compounds useful in the compositions and methods of treatment of the instant invention are other known CCK antagonists. They are CCK-B antagonists and mixed CCK-A/-B antagonists, the B component of which renders the compounds useful in the compositions and treatments of the instant invention.

Thienodiazepine compounds of formula 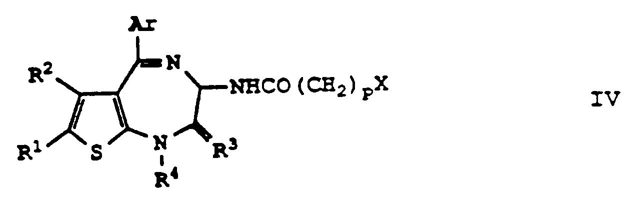
or a pharmaceutically acceptable salt thereof wherein
- R¹ and R²: are the same or different and respectively represent a hydrogen, a halogen, a C₁₋₂₀alkyl or an aralkyl selected from the group consisting of benzyl, 1-phenylethyl, 2-phenylethyl, 3-phenylpropyl, 4-phenylbutyl, 1-naphthylmethyl, and 2-naphthylmethyl unsubstituted or substituted on the aromatic ring by one to three substituent(s) selected from the group consisting of halogen, alkyl, alkoxy, trifluoromethyl, nitro, amino, cyano, and hydroxy,
or wherein R¹ and R² together form a ring selected from the group consisting of a cyclopentene ring, a cyclopentadiene ring, a cyclohexene ring, a cyclohexadiene ring, a benzene ring, a cycloheptene ring, a cycloheptadiene ring, and a cycloheptatriene ring;
- R³: represents an oxygen,
- R⁴: represent a hydrogen, a C₁₋₂₀alkyl, a C₂₋₈alkenyl, or a group of the formula -(CH₂)ₘCOOR⁶ (wherein R⁶ represents a hydrogen atom, a C₁₋₂₀alkyl, a C₂₋₈alkenyl, or an aralkyl selected from the group consisting of benzyl, 1-phenylethyl, 2-phenylethyl, 3-phenylpropyl, 4-phenylbutyl, 1-naphthylmethyl, and 2-naphthylmethyl unsubstituted or substituted on the aromatic ring by one to three substituent(s) selected from the group consisting of halogen, alkyl, alkoxy, trifluoromethyl, nitro, amino, cyano, and hydroxy, and m represents an integer of 1 to 6),
or R³ and R⁴ together form a group of the formula =N-N=C(R⁵)-,
wherein R⁵ represents a hydrogen, a C₁₋₂₀alkyl, a C₂₋₈alkenyl, an aralkyl selected from the group consisting of benzyl, 1-phenylethyl, 2-phenylethyl, 3-phenylpropyl, 4-phenylbutyl, 1-naphthylmethyl, and 2-naphthylmethyl unsubstituted or substituted on the aromatic ring by one to three substituent(s) selected from the group consisting of halogen, alkyl, alkoxy, trifluoromethyl, nitro, amino, cyano, and hydroxy, or a group of the formula -(CH₂)ₙCOOR⁷ (wherein R⁷ represents a hydrogen, a C₁₋₂₀alkyl, a C₂₋₈alkenyl, or an aralkyl selected from the group consisting of benzyl, 1-phenylethyl, 2-phenylethyl, 3-phenylpropyl, 4-phenylbutyl, 1-naphthylmethyl, and 2-naphthylmethyl unsubstituted or substituted on the aromatic ring by one to three substituent(s) selected from the group consisting of halogen, alkyl, alkoxy, trifluoromethyl, nitro, amino, cyano, and hydroxy, and n represents an integer of 1 to 6);
Ar and X are the same or different and respectively represent an aryl selected from the group consisting of a phenyl, a phenyl substituted by 1 or 2 substituents selected from the group consisting of halogen, alkyl, alkoxy, and nitro, and a 2-naphthyl or a heteroaryl selected from the group consisting of a pyridyl, an indolyl, a thienyl, a benzofuranyl, a 1H-benzimidazol-2-yl, and a 2-benzothiazolyl; and p represents an integer of 1 to 6, or its salt.

Preferred compounds of Formula IV above are
4-(2-chlorophenyl)-2-ethyl-6-(2-indole-carboxamido)-9-methyl-6H-thieno[3,2-f][1,2,4]-triazolo[4,3-a)[1,4]diazepine,
4-(2-chlorophenyl)-2-ethyl-6-(2-naphthalenecarboxamido)-9-propyl-6H-thieno[3,2-f][1,2,4]-triazolo[4,3-a][1,4]diazepine,
4-(2-chlorophenyl)-2-ethyl-6-(2-indolecarboxamido)-9-propyl-6H-thieno[3,2-f][1,2,4]-triazolo[4,3-a][1,4]diazepine,
4-(2-chlorophenyl)-2-ethyl-6-(3-indoleacetamido)-9-methyl-6H-thienyo[3,2-f][1,2,4]-triazolo[4,3-a][1,4]diazepine,
4-(2-chlorophenyl)-2-ethyl-6-(2-indolecarboxamido)-8-methyl-8H-6,7-dihydro-thieno-[3,2-f][1,4]diazepin-7-one,
4-(2-chlorophenyl)-2-ethyl-6-(3,4-dichlorobenzoylamino)-8-methyl-8H-6,7-dihydro-thieno-[3,2-f][1,4]diazepin-7-one,
4-(2-chlorophenyl)-2-ethyl-6-(2-indolecarboxamido)-6H-thieno-[3,2-f][1,2,4]triazolo-[4,3-a][1,4]diazepine,
6-(2-chlorophenyl)-7,8,9,10-tetrahydro-4-(2-indolecarboxamido)-1-methyl-4H-(1)benzothieno-[3,2-f][1,2,4]triazolo[4,3-a][1,4]diazepine, and
4-(2-chlorophenyl)-2-ethyl-6-(3,4-dichlorobenzoylamino)-9-methyl-6H-thieno[3,2-f][1,2,4]-triazolo[4,3-a][1,4]diazepine.

The above compounds and methods of preparing them are fully described in United States Patent 4,992,437, which is hereby incorporated by reference. Disclosed utilities are agents acting on the central and peripheral nervous systems for pancreatic disorders and gastrointestinal ulcers.

4H-pyrrolo-(1,2-a)thieno(3,2-f)(1,4-diazepine derivatives of formula 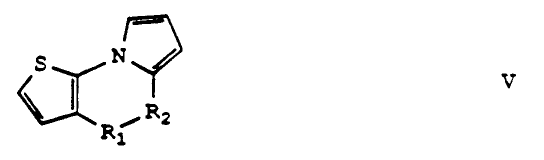
or a pharmaceutically acceptable salt thereof wherein are described in published European Application 446133. The utilities disclosed are for the treatment of cerebral accident and in ischemic syndrome.

R₁ is 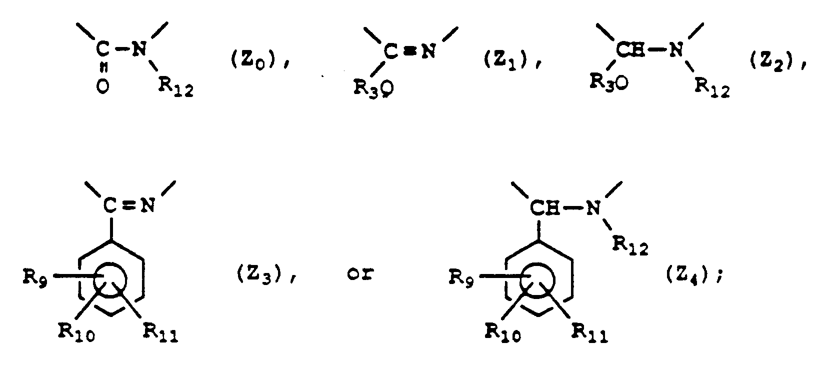
R₂ is methylene, hydroxymethylene, carbonyl, 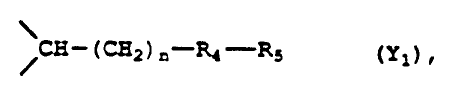 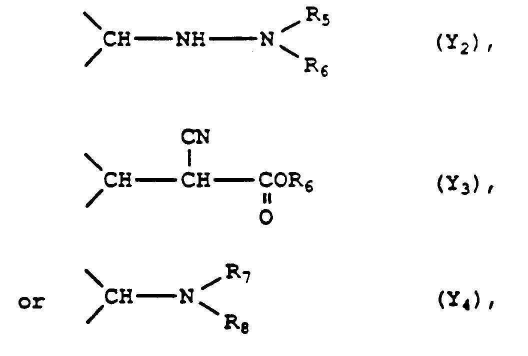
or R₂ with R₁₂ forms a radical of formula 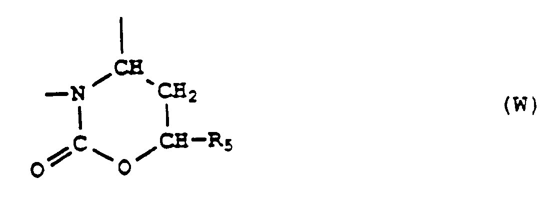
wherein R₅ is hydrogen, a straight or branched alkyl of from one to six carbon atoms, or R₁₂ is hydrogen;
- R₄ is: oxygen, sulfur, carbonyl, 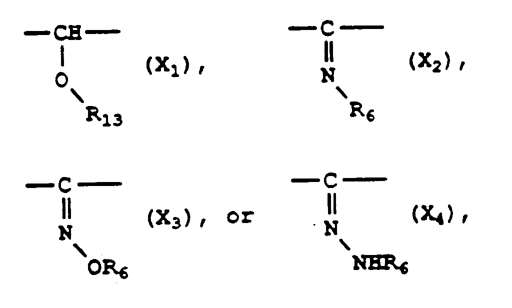
- n is: an integer of from zero to four;
- R₅ is: hydrogen, straight or branched alkyl of from one to ten carbon atoms optionally interrupted by one or more atoms of oxygen or sulfur, phenyl, benzoyl, aralkyl of from seven to ten carbon atoms which is unsubstituted or substituted by halogen, alkyl, nitro, alkoxy, carboxylalkyl, alkoxycarbonylalkyl, alkoxycarbonyl, a cyclic group, unsaturated from five to seven atoms containing at least one sulfur, oxygen, pyridinylcarbonyl, pyrimidylcarbonyl, clofibroyl, or 6-hydroxy 2,5,7,8-tetramethyl chroman-2-carbonyl;
- R₆ is: hydrogen or a straight or branched alkyl of from one to six carbon atoms;
- R₇ and R₈: are the same or different and are hydrogen, a straight or branched alkyl of from one to six carbon atoms, phenyl, phenylalkyl of seven to nine carbon atoms (optionally substituted on the aromatic portion by one or more atoms of halogen or an alkyl or alkoxy group of from one to six carbon atoms) or form a cyclic group of from five to seven atoms, saturated or unsaturated, containing one to two heteroatoms selected from oxygen, sulfur, and substituted by an alkylcarbonyl or alkoxycarbonyl of from two to five carbon atoms;
- R₉, R₁₀, and R₁₁: are the same or different and are hydrogen or an alkyl of from one to six carbon atoms;
- R₁₃ is: hydrogen, straight or branched alkylcarbonyl of from two to six carbon atoms, or benzoyl; with the provisos that when R₁ is Z₃ then R₂ is not methylene, when R₁ is Z₀ and R₁₂ is hydrogen then R₂ is not 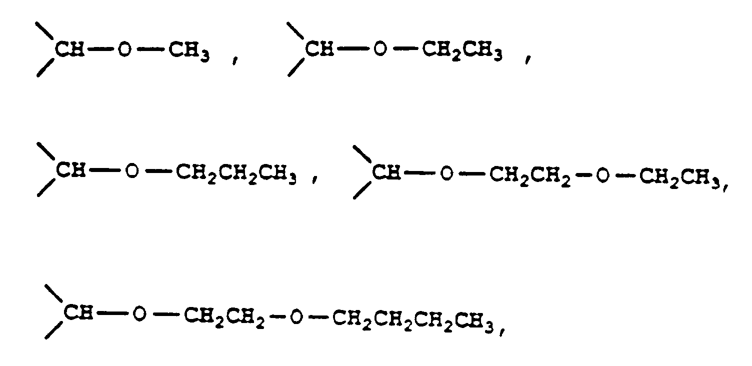 when R₁ is Z₀, R₁₂ is hydrogen and R₂ is Y₁, with n = 1 and R₄ is carbonyl then R₅ is not methyl, isopropyl, or phenyl, and when R₁ is Z₀ and R₁₂ is hydrogen, then R₂ is not carbonyl.

Compounds also useful in the instant invention are those of formula 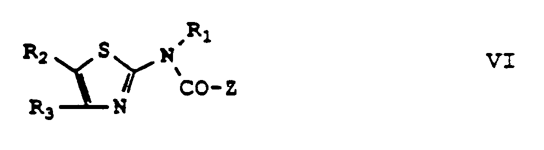
or a pharmaceutically acceptable salt thereof wherein
- R₁ is: H, 1-4Calkyl, phenyl-1-3C alkyl, -Z₁-NR₄R₅, -Z₂COOR₆, 2-5C cyano alkyl, or -Z₃-CONR₇R₈, 2-6C hydroxyalkyl, or 2-10C alkoxyalkyl;
- Z₁ is: 2-4C alkylene;
- Z₂ and Z₃: are 1-4C alkylene;
- R₄, R₅, R₇, R₈: are H or 1-4C alkyl, or
- R₄NR₅ or R₇NR₈: are morpholino, pyrrolidinyl, piperidino, piperazinyl, or 4-(1-3C alkyl)piperazinyl;
- R₆ is: H or 1-6C alkyl;
- R₂ is: H or 1-4C alkyl;
- R₃ is: 5-8C cycloalkyl optionally substituted by one or more 1-4C alkyl, aromatic group, e.g., phenyl (optionally substituted by one or more halo, 1-6C alkyl, 1-3C alkoxy, or thioalkoxy), NO₂, CF₃, or a heterocycle containing O, S, or N; or
- R₃ is: furyl, thienyl, pyrrolyl, pyrazolyl, imidazolyl, pyridyl, pyrazinyl, oxazolyl, or thiazolyl (optionally substituted by 1-3C alkyl or halo), or
- R₁ + R₃: is a group of formula (a) fixed with the carbon atom of the phenyl attached to position 4 of the thiazole; 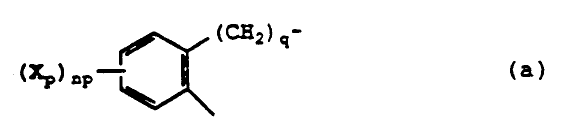
- q is: 1 to 4;
- Xₚ is: halo, 1-3C alkyl or alkoxy, NO₂, or CF₃;
- np is: 0 to 3;
- z is: heterocycle containing ≧1, O, S, or N condensed with an aromatic ring optionally containing one hetero atom and optionally substituted by >1 1-3C alkyl or alkoxy, benzyloxy, NO₂, NH₂, or CF₃.

These compounds are fully described in European Application 432040. They are disclosed as being useful in the treatment of colitis, pancreatitis, pancreatic carcinomas, gastric ulcers, Zollinger Ellison Syndrome, hyperplasia of the gastric cells, certain cancers, and for regulating appetite.

Other compounds, glutamic acid derivatives, are useful in the instant invention. They are disclosed in published European Application 383690. They are of formula 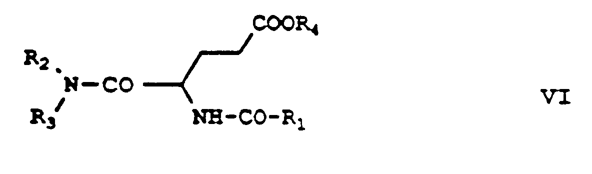
or a pharmaceutically acceptable salt thereof wherein
- R₁ is: aryl of up to 14C atoms (optionally substituted by one or more halogens, alkyl, or alkoxy groups of up to 8C, dialkylamino of up to 16C, CF₃, CN, or NO₂), indolyl, pyridyl, piperidinyl, quinolyl, thiazolyl, aralkyl of up to 18C (optionally substituted on the aryl by one or more of the groups listed above as possible substituents when R₁ is aryl),
- R₂ is: a group Ar-CH-Ar; or a group of formula (i): 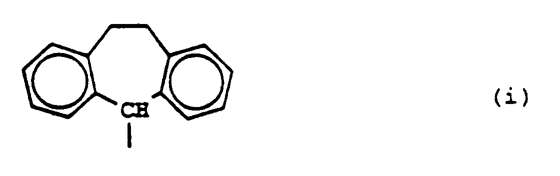
- Ar, Ar': is aryl of up to 14C (optionally substituted as indicated above), pyridyl, indolyl, or thiazolyl;
the two benzo rings in group (i) can be substituted as indicated for R₁;
- R₃ is: H; or
- N-R₂R₃ is: a 5-, 6-, or 7-membered heterocycle which may contain an O atom and may contain a second N atom, and which may be substituted by one or two alkyl groups of up to 8C, aryl of up to 14C, or (Ar)(Ar')CH-;
- R₄ is: H (optionally cyclic), alkyl of up to 8C, aralkyl of up to 18C (optionally substituted by any of the substituents listed above for R₁), or group (ii): 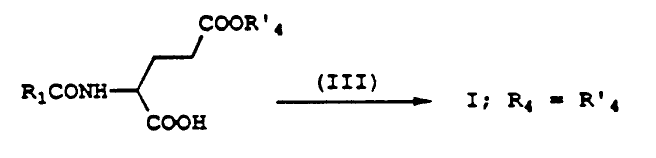
Compounds of Formula VI which are preferred are:
(S)-4-[[(1H-indol-2-yl)carbonylamino] 5-[[bis(4-methylphenyl)methyl]amino] 5-oxo pentanoic acid;
(S)-4-[[(1H-indol-2-yl)carbonyl]amino] 5-[(2-methoxyphenyl)phenyl methylamino] 5-oxo-pentanoic;
(S)-5-[[bis(4-chlorophenyl)methyl]amino] 5-oxo-4-[[(1H-indol-2-yl)carbonyl]amino]pentanoic acid; and
(S)-5-[(10,11-dihydrobenzo[a,d]cyclohepten-5-yl)amino 4-[[(1H-indol-2-yl)carbonyl]amino] 5-oxo-pentanoic acid.

The compounds are described as having utility in treating behavioral disorders.

Other compounds useful in the instant invention are pyrazolidinones of formula 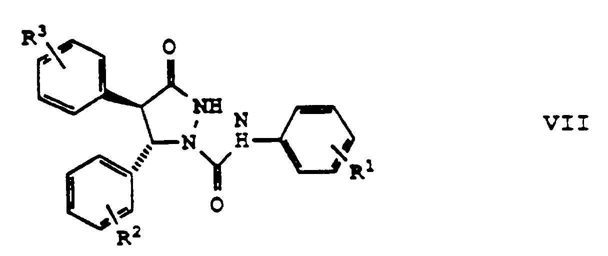
or a pharmaceutically acceptable salt thereof wherein
- R¹ is: 2,3-dichloro,
3,4-(CH₂)₄,
4-CF₃, or
4-Br;
- R² is: hydrogen,
2-chloro,
2,3-dichloro, or
CN; and
- R³ is: hydrogen -trans or -cis.

These are disclosed in Drugs of the Future 16(7):631-740 (1991). The compounds are made as described in Synthetic Examples 1 and 2 below.

Other compounds useful in the compositions and methods of treatment of the instant invention and quinazolinones disclosed in J. Med. Chem. 34:1505-1508 (1991) of formula 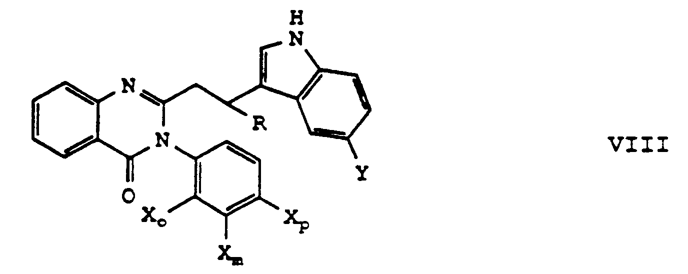
or a pharmaceutically acceptable salt thereof
wherein
- X₀ is: hydrogen, fluorine, chlorine, methoxy, or trifluoromethyl;
- Xₙ is: hydrogen, fluorine, chlorine, bromine, methyl ethyl isopropyl, methoxy, trifluoromethyl, propoxy, isopropoxy, cyclopentyloxyl, MeS, or NMe₂;
- Xₚ is: hydrogen, methoxy, ethoxy, isopropoxy, isopropyl, MeS, or NMe₂; or Xₘ and Xₚ together are -OCH₂O-;
- Y is: hydrogen, methyl, methoxy, fluorine, chlorine, or bromine; and
- R is: hydrogen or methyl.

A preferred compound is 2-[2-(5-bromo-1H-indol-3-yl)ethyl]-3-[3-(1-methylethoxy)phenyl]-4(3H)-quinazolinone.

Although several synthetic routes are available for preparing the above series, the following experimental procedure for synthesizing a compound is representative: 3-nitrophenol (50.0 g, 360 mmol), isopropyl iodide (76.19 g, 450 mmol), and K₂CO₃ (60 g) were combined and heated at reflux under N₂ overnight in acetone (400 mL). After solvent removal in vacuo, the residue was partitioned between EtOAc and H₂O. The separated organic layer was washed with 1 N NaOH, brine, dried over Na₂SO₄, and concentrated in vacuo to provide 56 g (86%) of 3-isopropoxynitrobenzene as a clear yellow oil.

A mixture of the above product (8.5 g, 50 mmol), PtO₂ (0.3 g), and EtOH (200 mL) was hydrogenated (40 psi H₂) at room temperature for 1.5 hours in a Paar shaker. The mixture was filtered through Celite and concentrated in vacuo to furnish 7.08 g of the desired aniline. This material was combined with isatoic anhydride (7.35 g, 45 mmol) and heated at 90°C for 2 hours. Upon cooling and addition of hexanes, the product crystallized to give 10.19 g (83%) of 2-amino-N-(3-isopropoxyphenyl)benzamide as a white solid. An analytical sample was obtained by recrystallisation from 20% EtOAc/hexanes, mp 79-86°C; ¹H NMR (CDCl₃) δ 1.36 (6H, d, J=6.1 Hz), 4.59 (1H, h, J=6.1 Hz), 5.2 (2H, bs), 6.6-6.8 (3H, m), 7.0-7.1 (1H, m), 7.2-7.4 (3H, m), 7.47 (1H, d, J=7.7 Hz), 7.80 (1H, bs); IR (CHCl₃) 1664, 1611, 1524, 1490 cm⁻¹; MS (FD) 270 (M⁺). Anal. (C₁₆H₁₈N₂O₂) C, H, N.

A solution of 3-[(2,2-dimethyl-4,6-dioxo-1,3-dioxan-5-yl)methyl)-5-bromoindole (4.12 g, 12 mmol) prepared according to the method of Farlow, et al (Farlow, D. S.; Flaugh, M. E.; Horvath, S. D.; Lavignino, E. R.; Pranc, P. Two Efficient Syntheses of Indole-3-Propionic Esters and Acids. Further Applications of Meldrum's Acid. Org. Prep. Proced. Int. 13:39-48 (1981), the above benzamide (3.48 g, 13 mmol) and pyridinium p-toluenesulfonate (1.64 g, 6.5 mmol) in 50 mL of pyridine was heated at reflux for 3.5 days. The reaction mixture was concentrated in vacuo, chromatographed (SiO₂, 30% EtOAc/hexanes), and crystallized to give 2.13 g (36%) of compound 22, mp 179-181°C; ¹H NMR (CDCl₃) δ 1.31 (3H, d, J=6.0 Hz), 1.34 (3H, d, J=6.1 Hz), 2.8 (2H, m), 3.2 (2H, m), 4.53 (1H, h, J=6.0 Hz), 6.7-7.6 (9H, m), 7.8 (2H, m), 8.2-8.4 (2H, m); IR (KBr) 1671 cm⁻¹; MS (FAB) 502, 504 (M⁺ + H). Anal. (C₂₇H₂₄N₃O₂Br) C, H, N.

The instant invention also covers at least 0.05 mg/kg to about 50 mg/kg of a compound, as defined above, present in the composition.

The instant invention also relates to a composition which is in oral dosage form.

The instant invention also relates to a method for treating psychoses, especially schizophrenia, in a mammal in need of such treatment which comprises administering a pharmaceutical composition of the instant invention in unit dosage form to said mammal.

The instant invention also relates to a method for treating anxiety in a mammal in need of such treatment which comprises administering a pharmaceutical composition of the instant invention in unit dosage form to said mammal.

The instant invention also relates to a method for treating the withdrawal response produced by chronic treatment followed by withdrawal of diazepam, nicotine, alcohol, or cocaine.

The compounds used in the invention may contain asymmetric carbon atoms. The invention includes the use of diastereomers, mixtures of diastereomers, or the use of mixed or the individual optical enantiomers. The invention includes all such forms of the compounds.

The compounds used in the instant invention include solvates, hydrates, and pharmaceutically acceptable salts thereof.

The pharmaceutically acceptable salts of the compounds used in the present invention include conventional nontoxic salts or quaternary ammonium salts.

The usefulness of the compounds of the instant invention as agents for treating psychoses and/or anxiety or withdrawal symptoms is demonstrated in the following pharmacological test procedure.

### METHODS

The compounds of the instant invention are useful as anxiolytic agents as described and discussed below.

Figure I illustrates the effectiveness of orally administered compound L-365,260. Figure II illustrates the effectiveness of orally administered compound LY-262690. Anxiolytic activity was assessed in the light/dark exploration test in the mouse (B. J. Jones, et al, Br. J. Pharmacol. 93:985-993, 1988).

Generally the number of mice used was 5 and the pretreatment time was about 40 minutes. The compound was given PO in 1- and 10-mg/kg doses.

The apparatus was an open-topped box, 45 cm long, 27 cm wide, and 27 cm high, divided into a small (2/5) area and a large (3/5) area by a partition that extended 20 cm above the walls. There was a 7.5 x 7.5 cm opening in the partition at floor level. The small compartment was painted black and the large compartment white. The floor of each compartment was marked into 9 cm squares. The white compartment was illuminated by a 100-watt tungsten bulb 17 cm above the box and the black compartment by a similarly placed 60-watt red bulb. The laboratory was illuminated with red light.

All tests were performed between 13 hundred hours, 0 minutes, and 18 hundred hours, 0 minutes. Each mouse was tested by placing it in the center of the white area and allowing it to explore the novel environment for 5 minutes. Its behavior was recorded on videotape and the behavioral analysis was performed subsequently from the recording. Five parameters were measured: the latency to entry into the dark compartment, the time spent in each area, the number of transitions between compartments, the number of lines crossed in each compartment, and the number of rears in each compartment.

In this test an increase in the time spent in the white area is a sensitive measure of the anxiolytic effects of several standard anxiolytic drugs. Drugs were dissolved in water or saline and administered either subcutaneously, intraperitoneally, or by mouth (PO) via a stomach needle.

Drugs such as alcohol, cocaine, diazepam, and nicotine can also be used in the light/dark exploration test in the mouse. For example, alcohol can be given in the drinking water as an 8% w/v solution for 14 days. After a 24-hour withdrawal period, the withdrawal response may be blocked by a composition of the instant invention when administered 10 mg/kg IP twice daily.

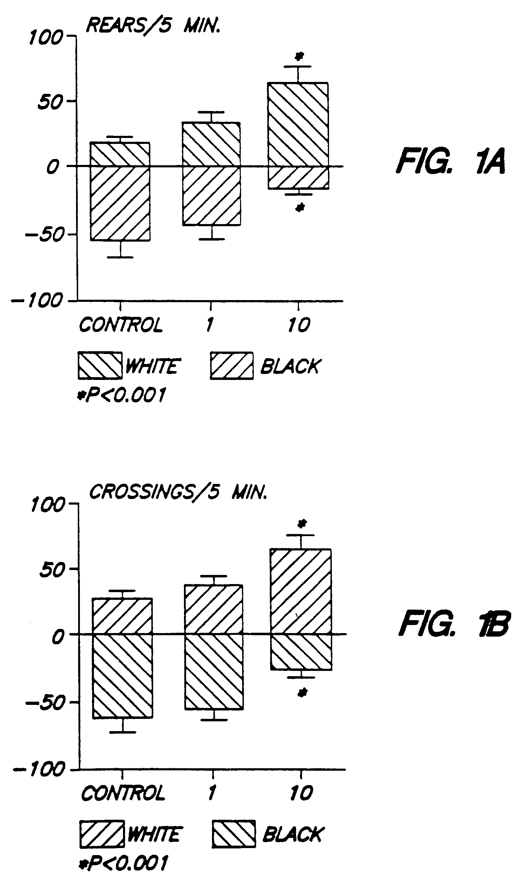

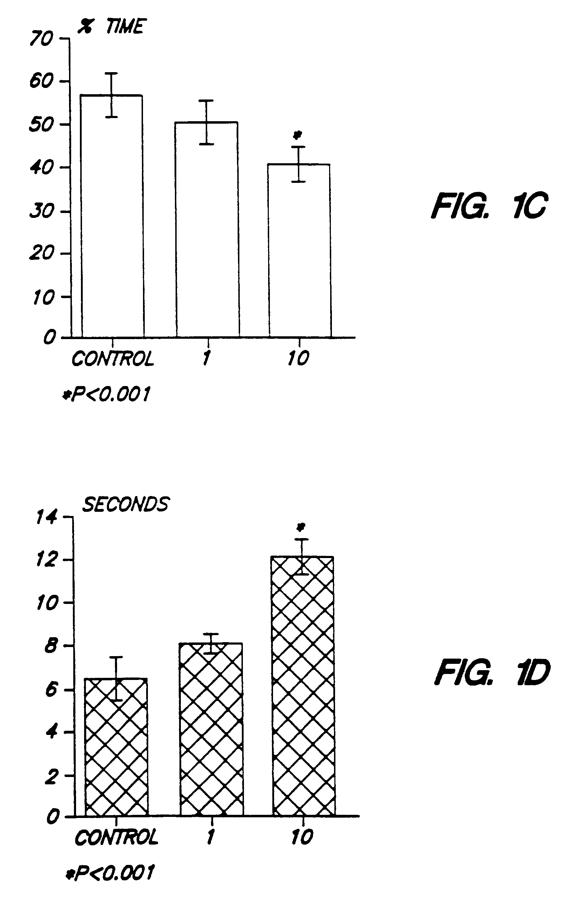

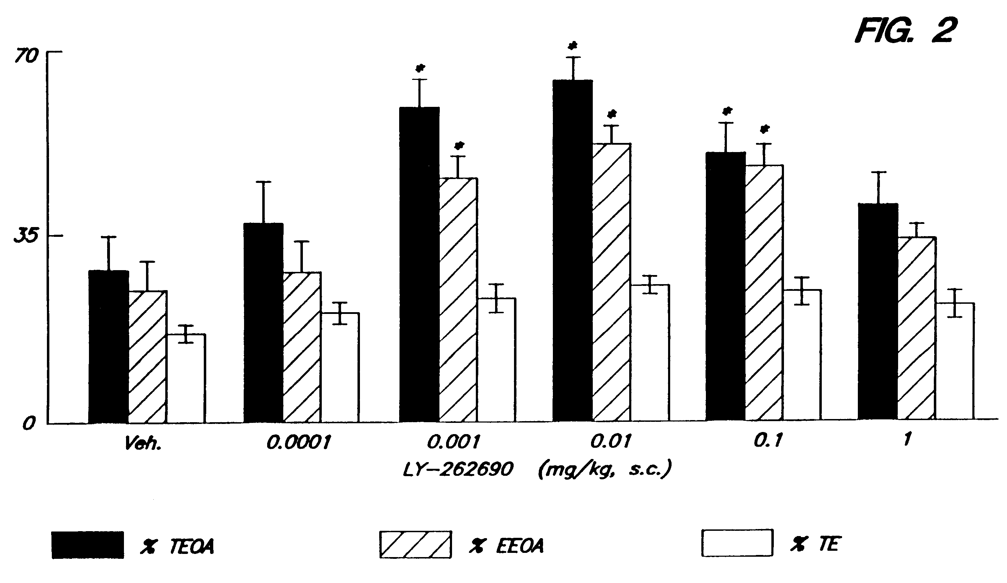

The results obtained with L-365,260 are shown in Figure 1. Control mice, not treated with L-365,260, but subjected to the aversive stimulus, showed a high level of rearing and line crossing activity in the black compartment compared to the white compartment. Treatment with 10 mg/kg L-365,260 orally reversed this response and the mice now showed higher rearing and line crossing activity in the white compartment than in the black compartment. Figure 1 also shows that L-365,260 reduced the total time spent by the mice in the black compartment and increased the time latency of the mice moving from the white side to the black side. These results show that L-365,260 possesses anxiolytic activity in this test.

The results in Figure II show that LY 262690 possesses anxiolytic activity.

The compositions of the instant invention are also useful as antipsychotic agents. The compounds of the instant composition were tested for their ability to reduce the effects of intra-accumbens amphetamine in the rat as described hereinafter.

Male Sprague Dawley (CD) Bradford strain rats were used. The rats were housed in groups of five at a temperature of 21 ± 2°C on a 12-hour light-dark cycle of lights-on between 07 hours 00 minutes and 20 hours 00 minutes. Rats were fed CRM diet (Labsure) and allowed water ad libitum.

Rats were anesthetized with chloral hydrate (400 mg/kg⁻¹ SC) and placed in a Kopf stereotaxic frame. Chronically indwelling guide cannulae (constructed of stainless steel tubing 0.6 mm diameter held bilaterally in Parspex holders) were implanted using standard stereotaxic techniques to terminate 3.5 mm above the center of the nucleus accumbens (Ant. 9.4, Vert. 0.0, Lat. 1.6) or 5.0 mm above the central nucleus of the amygdala (Ant. 5.8, Vert. -1.8, Lat. ±4.5) (atlas of De Groot, 1959). The guides were kept patent during a 14-day recovery period using stainless steel stylets, 0.3 mm diameter, which extended 0.5 mm beyond the guide tips.

Rats were manually restrained and the stylets removed. Intracerebral injection cannulae, 0.3 mm diameter, were inserted and drugs delivered in a volume of 0.5 µL over 5 seconds (a further 55 seconds was allowed for deposition) from Hamilton syringes attached via polythene tubing to the injection units. Animals were used on a single occasion only.

Behavioral experiments were conducted between 07 hours 30 minutes and 21 hours 30 minutes in a diet room maintained at 22 ± 2°C. Rats were taken from the holding room and allowed 1 hour to adapt to the new environment. Locomotor activity was assessed in individual screened Perspex cages (25 x 15 x 15 cm high) (banked in groups of 30), each fitted with one photocell unit along the longer axis 3.5 cm from the side; this position has been found to minimize spurious activity counts due to, for example, preening and head movements when the animal is stationary. Interruptions of the light beam were recorded every 5 minutes. At this time animals were also observed for the presence of any nonspecific change in locomotor activity, e.g., sedation, prostration, stereotyped movements, that could interfere with the recording of locomotor activity.

The abilities of the compounds to inhibit the hyperactivity caused by the injection of amphetamine into the nucleus accumbens of the rat was measured.

An increase in locomotor activity followed the bilateral injection of amphetamine (20 µg) into the nucleus accumbens; peak hyperactivity (50 to 60 counts 5 minutes⁻¹) occurred 20 to 40 minutes after injection.

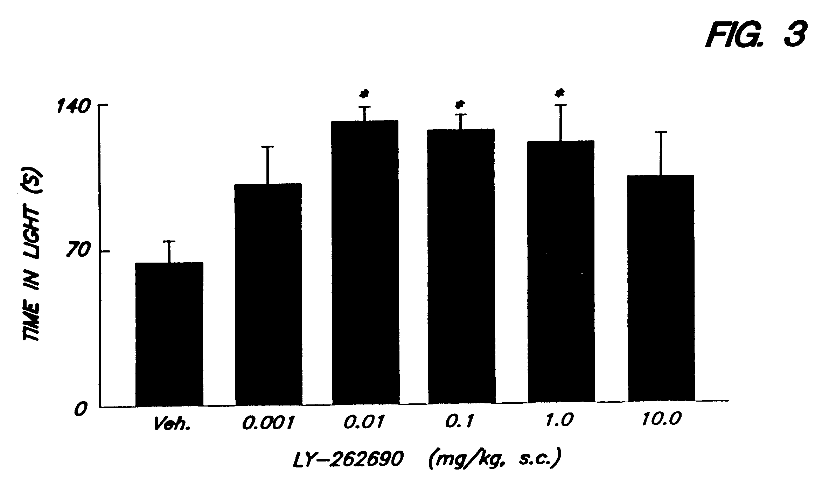

Intraperitoneal injection of the rats with compound (20A) (20 mg/kg or 30 mg/kg) or compound (20) (10 mg/kg) reduced the hyperactivity caused by the intra-accumbens injection of amphetamine (Figures 3 and 4). This test is known to be predictive of antipsychotic activity (Costall, Domeney, Naylor, and Tyers, Brit. J. Pharmac. 92:881-894).

Figure III shows the anxiolytic effects of compound in the Rat Elevated X-Maze Test on a dose range of 0.01 to 1.0 mg/kg SC. The anxiolytic effect is indicated by the time spent in the open arm end section compared with control C.

The original reference to the Elevated X-Maze is Handley and Mithani, Naunyn-Schmiedebergs Arch. Pharmacol. 327:1-5, 1984. The test used in the instant invention is the automated version as described by Field, Lewis, Lloyd, and Singh, Br. J. Pharmacol. (Suppl.)102:304, 1991.

The compound was first dissolved in 100% ethanol and serially diluted in alcohol. The required concentration was achieved by addition of 1.0% w/v carboxymethylcellulose, so that all solutions contained 10% ethanol.

The compound was administered subcutaneously (SC) 40 minutes before test, in a volume of 1 mL/kg.

Animals were examined on the Elevated X-Maze for 5 minutes. Results are shown in Figure III.

Percent time spent on end half sections of open arms (%TEOA), percent entries made onto end half sections of open arms (%TEOA) and total entries (TE).

The stars (*) show the groups which are significantly different from the vehicle (Veh.) treated group at the 0.05 level using ANOVA followed by Dunnett's t-test.

The compound increased both %TEOA and TEEAA, indicating an anxiolytic-like action. This appears to be a specific effect due to a lack of effect on total entries.

For preparing pharmaceutical compositions from compounds, inert, pharmaceutically acceptable carriers can be either solid or livid. Solid form preparations include powders, tablets, dispersible granules, capsules, cachets, and suppositories.

A solid carrier can be one or more substances which may also act as diluents, flavoring agents, solubilizers, lubricants, suspending agents, binders, or tablet disintegrating agents; it can also be an encapsulating material.

In powders, the carrier is a finely divided solid which is in a mixture with the finely divided active component. In tablets, the active component is mixed with the carrier having the necessary binding properties in suitable proportions and compacted in the shape and size desired.

For preparing suppository preparations, a low-melting wax such as a mixture of fatty acid glycerides and cocoa butter is first melted and the active ingredient is dispersed therein by, for example, stirring. The molten homogeneous mixture is then poured into convenient-sized molds and allowed to cool and solidify.

The powders and tablets preferably contain 5% to 70% of the active component. Suitable carriers are magnesium carbonate, magnesium stearate, talc, lactose, sugar, pectin, dextrin, starch, tragacanth, methyl cellulose, sodium carboxymethyl cellulose, a low-melting wax, cocoa better, and the like.

The term "preparation" is intended to include the formulation of the active component with encapsulating material as a carrier providing a capsule in which the active component (with or without other carriers) is surrounded by a carrier which is thus in association with it. Similarly, cachets are included.

Tablets, powders, cachets, and capsules can be used as solid dosage forms suitable for oral administration.

Liquid form preparations include solutions, suspensions, and emulsions. Sterile water or water-propylene glycol solutions of the active compounds may be mentioned as an example of liquid preparations suitable for parenteral administration. Liquid preparations can also be formulated in solution in aqueous polyethylene glycol solution.

Aqueous solutions for oral administration can be prepared by dissolving the active component in water and adding suitable colorants, flavoring agents, stabilizers, and thickening agents as desired. Aqueous suspensions for oral use can be made by dispersing the finely divided active component in water together with a viscous material such as natural synthetic gums, resins, methyl cellulose, sodium carboxymethyl cellulose, and other suspending agents known to the pharmaceutical formulation art.

Preferably the pharmaceutical preparation is in unit dosage form. In such form, the preparation is divided into unit doses containing appropriate quantities of the active component. The unit dosage form can be a packaged preparation, the package containing discrete quantities of the preparation, for example, packeted tablets, capsules, and powders in vials or ampoules. The unit dosage form can also be a capsule, cachet, or tablet itself, or it can be the appropriate number of any of these packaged forms.

When a compound of Formula I, IA, II, III, IV, V, VI, VII, or VIII is used in the instant invention in a human subject, the daily dosage will normally be determined by the prescribing physician with the dosage generally varying according to the age, weight, and response of the individual patient, as well as the severity of that patient's symptoms. However, in most instances, an effective daily dosage will be in the range of from about 0.05 mg/kg to about 50 mg/kg of body weight, and preferably, of from 0.5 mg/kg to about 20 mg/kg of body weight, administered in single or divided doses. In some cases, however, it may be necessary to use dosages outside these limits.

Examples of formulations of compounds or salts thereof of the instant invention are:

### EXAMPLE 1

### Injectables

Lorglumide with water for injection USP q.s.

The hydrochloride salt of the compound is dissolved in water and passed through a 0.2-micron filter. Aliquots of the filtered solution are added to ampoules or vials, sealed and sterilized.

### EXAMPLE 2

| Syrups 200 mg/5 mL syrup | |
|---|---|
| Compound | 12.5 g |
| Purified Water USP | 200 mL |
| Cherry Syrup qu | 1000 mL |

The compound is dissolved in water and to the resulting solution the syrup is added with mild stirring.

### EXAMPLE 3

| Capsules 50 mg, 100 mg, or 200 mg | |
|---|---|
| Compound 1 | 250 g |
| Lactose USP, Anhydrous q.s. or | 250 g |
| Sterotex Powder HM | 5 g |

Combine the compound and the lactose in a tumble blend for 2 minutes, blend for 1 minute with the intensifier bar, and then tumble blend again for 1 minute. A portion of the blend is then mixed with the Sterotex Powder, passed through a #30 screen, and added back to the remainder of the blend. The mixed ingredients are then blended for 1 minutes, blended with the intensifier bar for 30 seconds, and tumble blended for an additional minute. The appropriately sized capsules are filled with 141 mg, 352.5 mg, or 705 mg of the blend, respectively, for the 50 mg, 125 mg, and 250 mg containing capsules.

### EXAMPLE 4

| Tablets 50 mg, 100 mg, or 200 mg | |
|---|---|
| Corn Starch NF | 200 g |
| Cellulose, Microcrystalline | 46 g |
| Sterotex Powder HM | 4 g |
| Purified Water q.s. | 300 mL |

Combine the corn starch, the cellulose, and Compound 1 together in a planetary mixer and mix for 2 minutes. Add the water to this combination and mix for 1 minute. The resulting mix is spread on trays and dried in a hot air oven at 50°C until a moisture level of 1% to 2% is obtained. The dried mix is then milled with a Fitzmill through a #RH2B screen, and added back to the milled mixture and the total blended for 5 minutes by drum rolling. Compressed tablets of 150 mg, 375 mg, and 750 mg, respectively, of the total mix are formed with appropriate sized punches of the 50 mg, 125 mg, or 500 mg containing tablets.

### SYNTHETIC EXAMPLE 1

### Trans-5-(2-chlorophenyl)-3-oxo-4-phenyl-N-[4-(bromophenyl]-1-pyrazolidinecarboxamide

### Step 1. Preparation of αphenyl-2-chlorocinnamic acid

### Method used: Org. Syn. Coll. IV:777 (1963).

Phenylacetic acid (54.46 g, 0.4 M) was dissolved in acetic anhydride (80 mL). O-chlorobenzaldehyde (56.23 g, 0.4 M) was added slowly, with stirring. This was followed by the slow addition of triethylamine (40 mL). The reaction mixture was stirred at reflux for 5 hours. The reaction mixture was steam distilled until the distillate was no longer cloudy. The distillate was discarded. The aqueous residue was cooled. The solution was decanted from the gummy solid. This solid was dissolved in a 10% K₂CO₃ solution. The basic solution was charcoaled then filtered through a pooled Super cell. The filtrate was made acidic (pH 1) with 10% HCl, cooled, and the solid filtered. The product was recrystallized from 50% ethanol/H₂O to yield 52.14 g of white solid, mp 158-161°C.

### Step 2. Preparation of αphenyl-2-chlorocinnamic acid methyl ester

αPhenyl-2-chlorocinnamic acid (26.29 g (0.102 M) was dissolved in methanol (300 cc). Anhydrous HCl was bubbled through the reaction mixture with stirring for 15 minutes. The reaction mixture was refluxed for 2 hours, then HCl was bubbled through the reaction mixture for another 15 minutes. The reaction was stirred at reflux overnight. The methanol was removed in vacuo and the residue taken up in ether. The ether solution was washed with H₂O, saturated NaHCO₃ solution, and brine. It was then dried over MgSO₄. The ether solution was concentrated in vacuo to yield an oil that quickly solidified to yield 27.13 g of product, mp 67-69°C.

### Step 3. Preparation of 4-(O'-chlorophenyl)-5-phenyl-3-pyrazolidine

The ester from Step 2 (27.05 g, 0.0993 M) was dissolved in ethanol (75 cc). Eighty-five percent hydrazine hydrate (5.76 g, 0.0993 M) was added. The reaction mixture was stirred at reflux for 24 hours, then cooled. H₂O was added slowly with stirring. The product oiled out. The ethanol water was decanted from the oil. The oil was taken up in ether. The ether solution was washed with cold water, then dried over MgSO₄. The ether solution was concentrated in vacuo. A small amount of ether was added to the residue. The white solid was filtered and dried in vacuo to yield 9.56 g of product, mp 123-124°C.

### Step 4. Preparation of trans-5-(2-chlorophenyl)-3-oxo-4-phenyl-N-[4-(bromophenyl]-1-pyrazolidinecarboxamide

The pyrazolidone obtained in Step 3 (2.73 g, 0.01 M) was dissolved in THF (100 mL). p-Bromophenyl isocyanate (1.98 g, 0.01 M) was added. The reaction mixture was stirred overnight at room temperature. The clear solution was concentrated in vacuo to yield 4.73 g of a white solid. The solid was boiled in isopropyl ether. The insoluble solid was filtered from the warm ether and dried to yield 3.71 g of the product, mp 189-190°C.

| Analysis for C₂₂H₁₇BrClN₃O₂ (MW 470.762): | | | |
|---|---|---|---|
| Calcd.: | C, 56.13; | H, 3.64; | N, 8.91. |
| Found: | C, 56.43; | H, 3.87; | N, 8.71. |

IR NMR and MS consistent for the desired product.

### SYNTHETIC EXAMPLE 2

### Trans-5-(2-chlorophenyl)-3-oxo-4-phenyl-N-[4-(trifluoromethyl)phenyl]-1-pyrazolidinecarboxamide

Substituting αααtrifluoro-p-tolyl isocyanate (1.87 g, 0.01 M) for p-bromophenyl isocyanate in Step 4, one obtains 3.6 g of the product, mp 193-194°C.

| Analysis for C₂₃H₁₇ClF₃N₃O₂ (MW 459.859): | | | |
|---|---|---|---|
| Calcd.: | C, 60.07; | H, 3.73; | N, 9.14. |
| Found: | C, 60.16; | H, 3.81; | N, 9.09. |

IR, NMR and MS consistent for the desired product.

## Claims

1. A pharmaceutical composition for use in the treatment of psychoses and/or anxiety or in the treatment of the withdrawal response produced by chronic treatment followed by withdrawal of diazepam, nicotine, alcohol, or cocaine in a mammal, said composition comprising an antipsychotically or anxiolytically effective amount of at least one of the following CCK ligands:
pharmaceutically active derivatives of D,L-glutamic acid and D,L-aspartic acid of formulae: 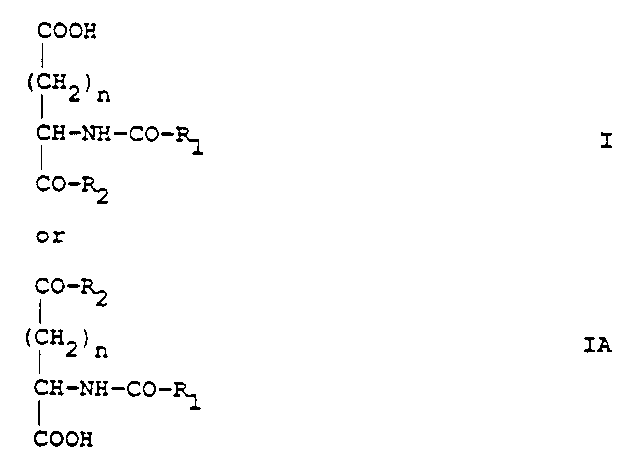 wherein n is 1 or 2;
R₁ is a phenyl group mono-, di-, or tri-substituted with linear or branched C₁-C₄ alkyl groups, which may be the same or different, or with halogens, with a cyano group or with a trifluoromethyl group;
R₂ is selected from the group consisting of morpholino, piperidino, and amino with one or two linear, branched, or cyclic alkyl group substituents containing from 1 to 8 carbon atoms which may be the same or different; or 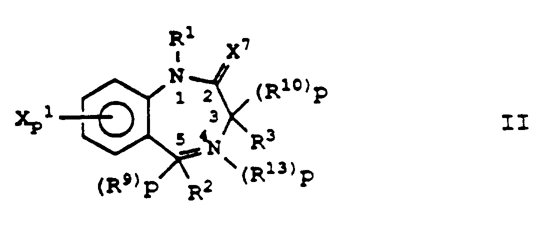
wherein
R¹ is H, C₁-C₆ linear or branched alkyl, loweralkenyl, lower alkynyl, -X¹²COOR⁶, -X¹¹cycloloweralkyl, -X¹²NR⁴R⁵, -X¹²CONR⁴R⁵, -X¹²CN, or -X¹¹CX₃¹⁰;
R² is H, loweralkyl, substituted or unsubstituted phenyl (wherein the substituents may be 1 or 2 of halo, loweralkyl, loweralkoxy, lowralkylthio, carboxyl, carboxyloweralkyl, nitro, -CF₃, or hydroxy), 2-, 3-, 4-pyridyl, 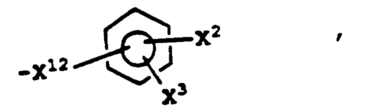 -X¹²SCH₃, -X¹²SOCH₃, -X¹²SO₂CH₃, or -X¹²COOR⁶;
R³ is -X¹¹R⁷, 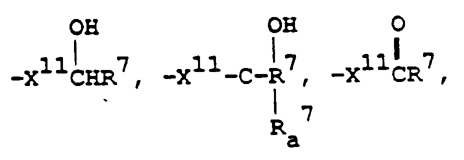 -X¹¹NR¹⁸(CH₂)_{q}R⁷, 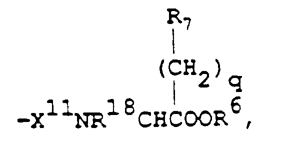 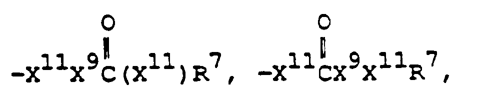 -NH(CH₂)₂₋₃NHR⁷, -NH(CH₂)₂₋₃NHCOR⁷, 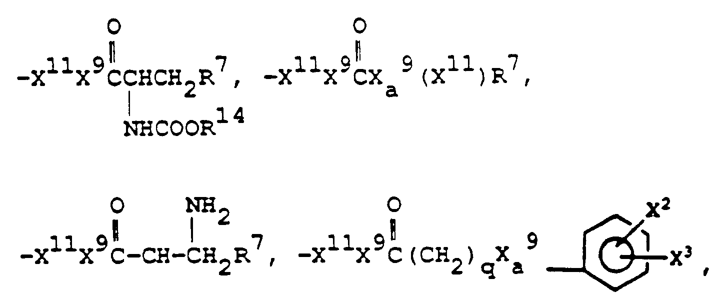 -X¹¹NR¹⁸SO₂(CH₂)_{q}R⁷ or 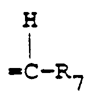
wherein
R₄ and R₅ are independently R⁶ or in combination with the N of the NR⁴R⁵ group form an unsubstituted or mono or disubstituted, saturated or unsaturated, 4-7-membered heterocyclic ring or benzofused 4-7-membered heterocyclic ring, or said heterocyclic ring or said benzofused heterocyclic ring which further comprises a second heteroatom selected from O and NCH₃ and the substituent(s) is/are independently selected from C₁₋₄alkyl;
R⁶ is H, loweralkyl, cycloloweralkyl, substituted or unsubstituted phenyl, or substituted or unsubstituted phenylloweralkyl wherein the phenyl or phenylloweralkyl substituents may be 1 or 2 of halo, lowralkyl, loweralkoxy, nitro, or CF₃;
R₇ and Rₐ⁷ are independently α- or β-naphthyl, substituted or unsubstituted phenyl (wherein the substituents may be 1 or 2 of halo, -NO₂, -OH, -X¹¹NR⁴R⁵, loweralkyl, CF₃, CN, SCF₃, C=CH, CH₂SCF₃, 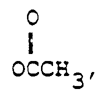 OCHF₂, SH, SPh, PO₃H-loweralkoxy, or loweralkylthio, COOH), 2-, 3-, 4-pyridyl, 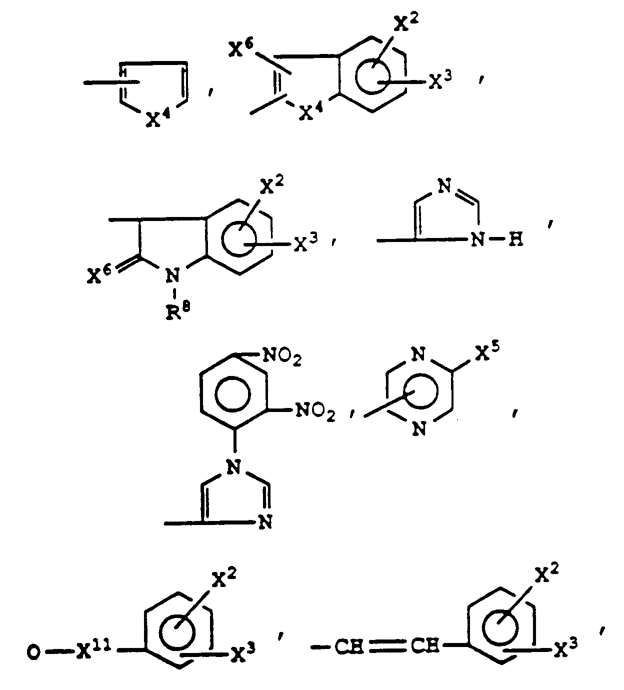 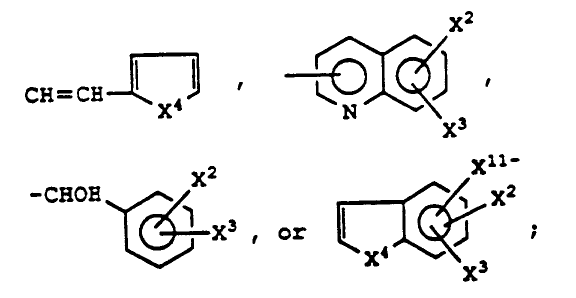
R⁸ is H, loweralkyl, cycloloweralkyl, -X¹²CONH₂, -X¹²COOR⁶, -X¹²-cycloloweralkyl, -X¹²NR⁴R⁵, 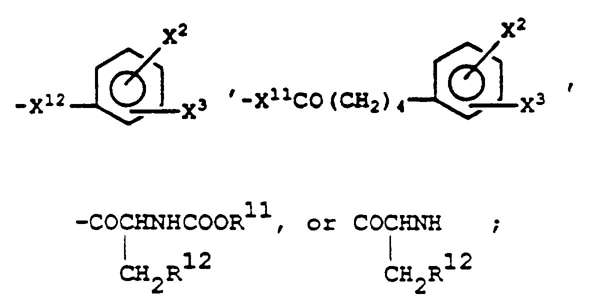
R⁹ and R¹⁰ are independently H, -OH, or -CH₃,
R¹¹ and R¹² are independently loweralkyl or cycloloweralkyl;
R¹³ is H, loweralkyl, acyl, O, or cycloloweralkyl;
R¹⁴ is loweralkyl or phenylloweralkyl;
R¹⁵ is H, loweralkyl, 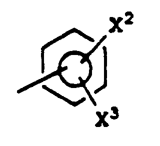 or -NH₂;
R¹⁸ is H, loweralkyl, or acyl;
p is 0 when its adjacent == is unsaturated and 1 when its adjacent == is saturated except that when R¹³ is O, p is 1 and == is unsaturated;
q is 0 to 4;
r is 1 or 2;
X¹ is H, -NO₂, CF₃, CN, OH, loweralkyl, halo, loweralkylthio, loweralkoxy, -X¹¹COOR⁶, or -X¹¹NR⁴R⁵-;
X² and X³ are independently H, -OH, -NO², halo, loweralkylthio, loweralkyl, or loweralkoxy;
X⁴ is S, O, CH₂, or NR¹⁸ or NR⁸;
X⁵ is H, CF₃, CN, -COOR⁶, NO₂, or halo;
X⁶ is O or HH;
X⁷ is O, S, HH, or NR¹⁵;
X⁸ is H or loweralkyl;
X⁹ and Xₐ⁹ are independently NR¹⁸ or O;
X¹⁰ is F, Cl, or Br;
X¹¹ is absent or C₁₋₄ linear or branched alkylidene;
X¹² is C₁₋₄ linear or branched alkylidene;
-- is a saturated or unsaturated bond; or 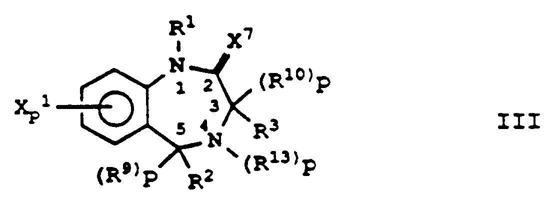
wherein R¹, R², R⁴, R⁵, R⁶, R⁸, R⁹, R¹⁰, R¹¹, R¹², R¹³, R¹⁴, R¹⁵, p, q, r, X¹, X², X³, X⁵, X⁶, X⁷, X⁸, X⁹, X¹⁰, X¹¹, and X¹² are as defined above,
R³ is -X¹¹NR¹⁸(CH₂)_{q}R¹⁶, 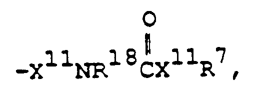 -NH(CH₂)₂₋₃NHR⁷, -NH(CH₂)₂₋₃NHCOR⁷, 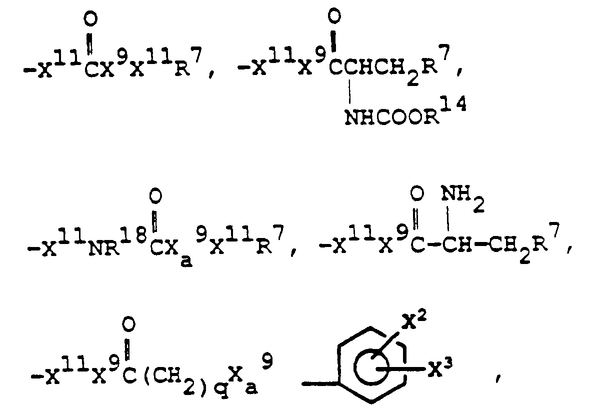 -X¹¹NR¹⁸SO₂(CH₂)_{q}R⁷ or 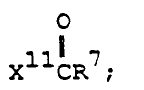
R⁷ is α- or β-naphthyl, substituted or unsubstituted phenyl (wherein the substituents may be 1 to 2 of halo, -NO², -OH, -X¹¹NR⁴R⁵, loweralkyl, CF₃, CN, SCF₃, 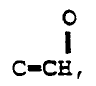 CH₂SCF₃, OCCH₃, OCHF₂, SH, SPh, PO₃H, loweralkoxy, loweralkylthio, or COOH), 2-, 3-, 4-pyridyl, 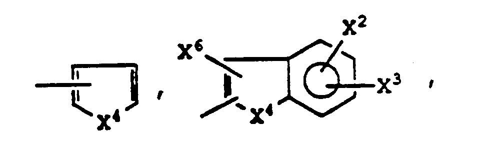 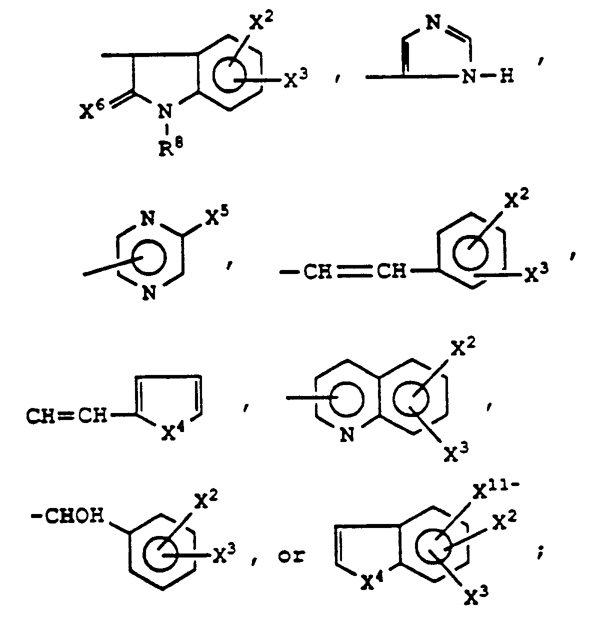 R¹⁶ is α- or β-naphthyl or 2-indolyl;
R¹⁸ is H or loweralkyl; and
= is a saturated or unsaturated bond or a pharmaceutically acceptable salt there of in combination with a pharmaceutically acceptable carrier.

2. A pharmaceutical composition according to Claim 1 comprising lorglumide, loxiglumide, L-364,718, L-365,260, or a pharmaceutically acceptable salt thereof.

3. A pharmaceutical composition according to Claim 1 characterized in that it contains as active ingredient a compound in amounts from about 0.05 mg/kg to about 50 mg/kg.

4. A pharmaceutical composition according to Claim 1, in oral dosage form.

5. A method for treating psychoses in a mammal in need of such treatment which comprises administering a pharmaceutical composition according to Claim 1 in unit dosage form.

6. A method for treating psychoses in a mammal in need of such treatment which comprises administering lorglumide or a pharmaceutically acceptable salt thereof in combination with a pharmaceutically acceptable carrier to said mammal.

7. A method of treating psychoses in a mammal in need of such treatment which comprises administering loxiglumide or a pharmaceutically acceptable salt thereof in combination with a pharmaceutically acceptable carrier to said mammal.

8. A method for treating psychoses in a mammal in need of such treatment which comprises administering L-364,718 or a pharmaceutically acceptable salt thereof in combination with a pharmaceutically acceptable carrier to said mammal.

9. A method for treating psychoses in a mammal in need of such treatment which comprises administering L-365,260 or a pharmaceutically acceptable salt thereof in combination with a pharmaceutically acceptable carrier to said mammal.

10. A method of treating anxiety in a mammal in need of such treatment which comprises administering a pharmaceutical composition according to Claim 1 in unit dosage form.

11. A method for treating anxiety in a mammal in need of such treatment which comprises administering lorglumide or a pharmaceutically acceptable salt thereof in combination with a pharmaceutically acceptable carrier to said mammal.

12. A method for treating anxiety in a mammal in need of such treatment which comprises administering L-364,718 or a pharmaceutically acceptable salt thereof in combination with a pharmaceutically acceptable carrier to said mammal.

13. A method for treating anxiety in a mammal in need of such treatment which comprises administering L-365,260 or a pharmaceutically acceptable salt thereof in combination with a pharmaceutically acceptable carrier to said mammal.

14. A method of treating anxiety in a mammal in need of such treatment which comprises administering loxiglumide or a pharmaceutically acceptable salt thereof in combination with a pharmaceutically acceptable carrier to said mammal.

15. A method for treating or preventing the withdrawal response produced by withdrawal of chronic treatment with diazepam in a mammal in need of such treatment which comprises administering a pharmaceutical composition according to Claim 1 in unit dosage form.

16. A method for treating or preventing the withdrawal response produced by withdrawal from chronic use of cocaine in a mammal in need of such treatment which comprises administering a pharmaceutical composition according to Claim 1 in unit dosage form.

17. A method for treating or preventing the withdrawal response produced by withdrawal from chronic use of alcohol in a mammal in need of such treatment which comprises administering a pharmaceutical composition according to Claim 1 in unit dosage form.

18. A method for treating or preventing the withdrawal response produced by withdrawal from chronic use of nicotine in a mammal in need of such treatment which comprises administering a pharmaceutical composition according to Claim 1 in unit dosage form.

19. A method for treating anxiety in a mammal in need of such treatment which comprises administering LY 262690 or a pharmaceutically acceptable salt thereof in combination with a pharmaceutically acceptable carrier to said mammal.

20. A method for treating anxiety in a mammal in need of ouch treatment which comprises administering 2-[2-(5-bromo-1H-indol-3-yl)ethyl]-3-[3-(1-methylethoxy)phenyl]-4(3H)-quinazolinone or a pharmaceutically acceptable salt thereof in combination with a pharmaceutically acceptable carrier to said mammal.

21. A method for treating anxiety in a mammal in need of such treatment which comprises administering LY 262691 or a pharmaceutically acceptable salt thereof in combination with a pharmaceutically acceptable carrier to said mammal.

22. A method for treating anxiety in a mammal in need of such treatment which comprises administering L-365091 or a pharmaceutically acceptable salt thereof in combination with a pharmaceutically acceptable carrier to said mammal.

23. A method for treating anxiety in a mammal in need of such treatment which comprises administering (S)-5-[(10,11-dihydrodibenzo-[a,d]cyclohepten-5-yl)amino]-4-[(1H-indol-2-yl)carbonyl]amino]-5-oxo-pentanoic acid, or a pharmaceutically acceptable salt thereof in combination with a pharmaceutically acceptable carrier to said mammal.

24. A method for treating anxiety in a mammal in need of such treatment which comprises administering 5,6-dihydro-4-oxo-4H-pyrrolo[1,2-a]thieno[3,2-f][1,4]-diazepin-6-yl)cyanoacetate or a pharmaceutically acceptable salt thereof in combination with a pharmaceutically acceptable carrier to said mammal.

25. A method for treating anxiety in a mammal in need of such treatment which comprises administering Virginiamycin or a pharmaceutically acceptable salt thereof in combination with a pharmaceutically acceptable carrier to said mammal.
